# EUROPEAN PATENT SPECIFICATION

(11) **EP 1 358 745 B1**
(45) Date of publication and mention of the grant of the patent: **10.12.2008**
(21) Application number: 01964298.2
(22) Date of filing: 22.08.2001
(51) Int. Cl.: H04L 29/06

(54) **MEDICAL DEVICE SYSTEMS IMPLEMENTED NETWORK SYSTEM FOR REMOTE PATIENT MANAGEMENT**
NETZWERKIMPLEMENTIERTE MEDIZINISCHE ANLAGESYSTEME FÜR FERNPATIENTENVERWALTUNG
SYSTEMES DE DISPOSITIFS MEDICAUX MIS EN OEUVRE PAR L'INTERMEDIAIRE D'UN SYSTEME DE RESEAU POUR SURVEILLER UN PATIENT A DISTANCE

(30) Priority: 22.08.2000 US 227164 P
(43) Date of publication of application: 05.11.2003
(73) Proprietor: MEDTRONIC, INC., Minneapolis MN 55432-5604 (US)
(72) Inventor: LINDEN, Gregory, Shorewood, MN 55331 (US); RIFF, Kenneth, M., Orono, MN 55391 (US)
(74) Representative: Hughes, Andrea Michelle
(86) International application number: PCT/US2001/026172
(87) International publication number: WO 2002/017593

(56) References cited:
- WO-A-00/10455
- WO-A-98/15910
- US-A- 5 619 991
- US-A- 5 915 240
- KATSIKAS S K D ET AL: "Using trusted third parties for secure telemedical applications over the WWW: The EUROMED-ETS approach" INTERNATIONAL JOURNAL OF MEDICAL INFORMATICS, ELSEVIER SCIENTIFIC PUBLISHERS, SHANNON, IR, vol. 49, no. 1, March 1998 (1998-03), pages 59-68, XP004149462 ISSN: 1386-5056

## Description

The invention relates to medical devices implemented and communicable through network systems such as the internet. More specifically, the invention relates to patients wearing implantable or externally mounted medical devices in which the devices are communicable to remote healthcare professionals.

### Background of the Invention

Medical devices such as cardiac systems, drug delivery systems, neurological products and similar ot her products are implanted in patients for various clinical reasons. Some of these devices may collect and document data on a continuous basis. However, the state of the art is currently to ask patients to see their doctors or other health professionals on a regular basis to retrieve and check the physiological data collected in these devices.

As medical devices become very sophisticated, in both reliability and maintainability, the need for patients to visit their doctors on a regular basis may not be required by coverage plans or for other rationale. Various attempts to remotely engage or monitor patients have been suggested, such as for example those described in United States Patent Nos 6,168,563 B1; 6,221,011 B1; 6,203,495 B1; 6,250,309 B1; 5,633,910; 5,752,976 and WO 98/15910 together with WO 00/10455. However, for patients with chronic disease, the management of the disease has become a critical aspect which affects both cost and the quality of life of the patient Accordingly, patients with implantable medical devices or externally mounted devices that monitor critical medical data are either kept in hospitals or the patients are required to visit their physicians on a very regular basis. However, a data transfer and review system that enables doctors and physicians to monitor patients on an as-needed basis and as frequently as possible, while allowing patients to stay at home, is a highly desirable service. Such a service would also enable the patient to have access to their own personal data by enabling real time data management and review by professionals as well as the patient Further, medical devices could be designed to enable patients to be interactive with the devices that are monitoring their physical and medical parameters such that the patient could also be involved in managing their disease on a day-to-day basis. More specifically, if patients are allowed to have access both to the operation of their device and reports that are stored in them, they may have sessions with their doctors and will also be well-informed in managing their disease, thereby becoming active partners in the management of their own disease.

### Summary of the Invention

The present invention provides an internet-based method for a service to connect a remote patient having an implanted medical device to a database network for medical device data exchange and review comprising:
providing a web-site having a user interface wherein the user interface includes a secure sign-in input to access the database network site;
receiving at the web-site inputs associated with a specific medical device and patient;
confirming the identity of the medical device and the patient;
reviewing data indicative of an event from said implanted medical device;
alerting a caregiver to said event; and
enabling said caregiver to access the database in a single sign-on action to use the service.

Preferred embodiments will now be described, by way of example only, with reference to the drawings.
Figure 1 is a schematic representation of a patient with exemplar implanted medical device components interfacing with a remote patient management network.
Figure 2 is a schematic diagram of the system and data flow of an embodiment of the invention.
Figure 3 is a schematic diagram of the system and data flow of an embodiment of the invention.
Figure 4 is a schematic diagram of the system and data flow of an embodiment of the invention.
Figure 5 is a decision making flow chart of a method of use of the invention.

Attempts to provide improved healthcare to patient populations using technological methods have met with varying degrees of success. Indeed, however, a very critical problem has emerged in most such efforts. The patients and healthcare providers alike risk increased isolation as the use of technology increases. This isolation and sense of de-personalization in the healthcare system is often a chief complaint of patients, as well as a source of potential conflict which could actually impede the formation of candid dialogue which is at the core of the best models of patient-physician interface.

Medtronic, Inc. has developed medical devices which are able to detect large amounts of valuable patient specific information and process that information so as to decide whether one or more specific applications of therapy or ongoing diagnosis is merited. This is technology that has emerged in application of implantable medical devices over the last several decades, and has transformed many lives due to its use by Medtronic and other companies. However, the inventors have now identified new and improved mechanisms by which the technical means of harnessing the ongoing and simultaneous revolutions in medical device technologies and information technologies to achieve a new level of patient and healthcare provider satisfaction, quality of care, improved service, improved efficiencies and improved economics is realized. This combination of new technical processes has overcome the combined technical problems associated with: sensing highly specific signals, parsing and validating high volumes of data, power management of implanted medical devices, bandwidth allocation to integrate all levels of a web-enabled network among multiple foreign users, timing of signals and processing, accessibility and security, display limitations and demands, data routing in multiple paths including wireless paths, partial user unfamiliarity with technology, partial user incapacity, and other challenges. The technical effect of the selected protocols along with the application of advanced information technology has resulted in a significant technical contribution to the art of integrated network-based signal and resource management for programmable and high relevance detected data signals.

Applicants have recognized that a remarkable innovation is achieved in deeply utilizing the now very robust data collection capabilities of various medical devices and integrating that information (either before, during or after analyzing or processing the information) into a data network. This causes integration of the patient and healthcare providers, or others, into a collaborative effort resulting in patient-specific healthcare improvement and system efficiencies. This is a pioneering effort in identifying a combination of historic problems, applying technical methodology to combine the best medical and information technologies resulting in a solution which transforms the way resources are allocated, which in turn leads to improvements for care of patients with various diseases and conditions, particularly those having chronic conditions.

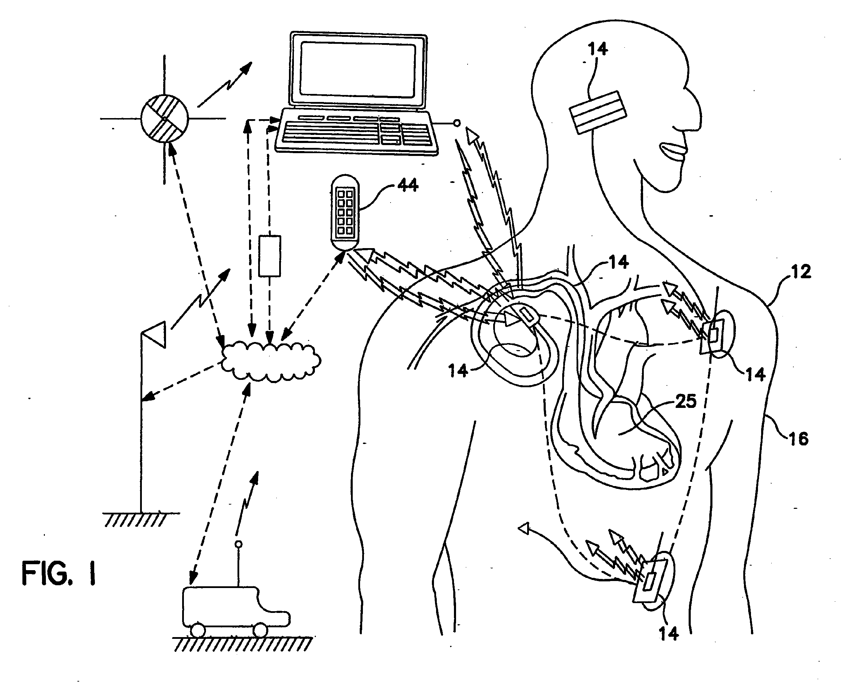

In one example, patients having advanced cardiovascular disease (and their care givers) may utilize this invention to great advantage. Using one or more medical devices 14, such as the implantable cardiac-related devices known as either the Medtronic Chronicle device or the GEM III DR device, shown in patient 20 of Figure 1, a patient's cardiac system is closely monitored. This figure also illustrates alternate examples of device 14 which includes neurologic device 14 and drug infusion device 14 as well. The invention may utilize a device such as those noted above which is implanted for sensing directly in a cardiac chamber 25, and which may have more data acquisition capability and processing power than any other known implantable on earth, or even a more limited capability device. Such a powerful data acquisition platform is able to store a wealth of information regarding cardiac and overall patient physiologic data in the onboard RAM of the device that can be uploaded through wireless technology, such as radio frequency (RF) telemetry, telemetry C, or the like, and which is representatively shown within lines 31 of Figure 1. Following acquisition of this medical data from inside the body, then advanced information and communication technologies are utilized to communicate this information to other locations or users.

In one embodiment, another device, shown in representative manner as device 44 in Figure 1, is arranged to be near the patient and to obtain the information from the implanted device and transmit the data to a remote network. Device 44 may be alternately described as a remote interrogator or by other terminology, which is not meant to limit its actual technical characteristics in any way, but rather to identify a data communication device for use with medical devices as generally described herein.

As shown in Figures 1-5, a secure transmission of the physiologic data from the patient then occurs, with the data flowing to a robust network where it can be stored, processed, analyzed and presented for viewing via a web browser or other interfaces. These technologies have been combined and improved with the specific purpose of providing a reliable, scalable, secure and accessible system for worldwide real-time use of the patient's data. This is an extraordinary, full cycle, breakthrough over known limited systems for disease and patient management Indeed, this invention re-organizes and prioritizes patient care in a manner not previously known along a new model of data and human interaction. In the cardiac example above, information about the patient's heart is online and accessible at all times. The system provides the physician and others with a continuous, longitudinal record of cardiac status rather than the snapshot that is received when the patient comes into a clinic. This is also real-time and continuous, rather than responsive and subject to any specific event or to known monitoring device availability or limitations. Physicians may then use this new system and methods to implement timely, systematic therapeutic regimens for their patients. It is recognized that either the virtual or the physical locations of certain elements of this system, and methods of performing the services and advantageous steps of the invention, may be optionally determined to occur in either one or more jurisdictions depending on the element, step, signal or advantage enabled. Accordingly, appropriate data use and other security authorizations or measures are fully contemplated, and may be relevant to judging the utilization and scope of challenges successfully overcome by this invention.

Using this invention, the patient, and optionally the patient's friends and family, becomes active participants in the management of the patient's health. This enables the patient to be empowered as a collaborator through the system's patient portal, where he or she can access individualized educational, monitoring and self-care programs at any time from virtually any location. Although a deep and rich variety of data is acquirable, data having the most relevance for the specific patient may be designated for sensing, e.g. a patient's monitored signals may include intracardiac pressures, heart rate, physical activity, or other signals. This process of continuous data collection is then supplemented by the uploads of all or some of the data to an external proximate device (i.e. in home, office or car) for transmission of the critical physiologic data securely to a healthcare management network. Physicians can access the network via a Web site at any time and review screens that present summary information from the latest upload, trend information accumulated over time, and/or detailed records from specified times or problem episodes.

The invention has the potential to transform the management of chronic diseases like heart failure and cardiac arrhythmias, neurologic conditions, or conditions requiring drug infusions, and other health needs relating to health conditions. It will improve the quality of care by providing the physicians with real-time data about their patients' physiological (and maybe other) conditions so that corrective actions may be taken in a timely manner, if appropriate. This invention will improve the quality of life for the patients, enabling them to remain in their homes or travel as desired without the same level of constant worry concerning an emergency hospitalization. The invention will result in dramatic lowering of the cost of managing chronic disease by reducing unnecessary hospitalizations and clinic visits. Perhaps most importantly, this system and methodology will help restore the close bond of the patient-physician relationship through the power of direct connection and the proliferation of high relevance understandable knowledge to all participants in the process. For example, the system and methodology makes personalized health information, even detailed information on the performance of a patient's own heart, available to the patient and loved ones (or other advocates of the patient) through an interactive web or mobile portal. Trials of this system have alrea dy observed these very positive effects, synergies and impacts.

In addition to the profound implications for quality of care, standard of care, and related issues in relation to this invention, the financial and business impact of this system and methodology is quite revolutionary. The system is designed to more successfully manage people with chronic diseases by leveraging the Internet or similar communication medium that allows continuous or near continuous real-time data access. The interaction with clinical researchers of users of this invention is also valuable. This degree of interaction allows for more rapid and in-depth clinical analysis of disease symptoms and treatments, thereby reducing the overall costs of such efforts. Such real- time access to patient data also facilitates administration of drug and other therapy in a more responsive and economical fashion. The involvement of the patient and the patient' s advocate(s) promotes improved communication and outcomes. Combining this with the known patient desire for greater convenience and a closer connection to the physician, then the advantages of this invention are many.

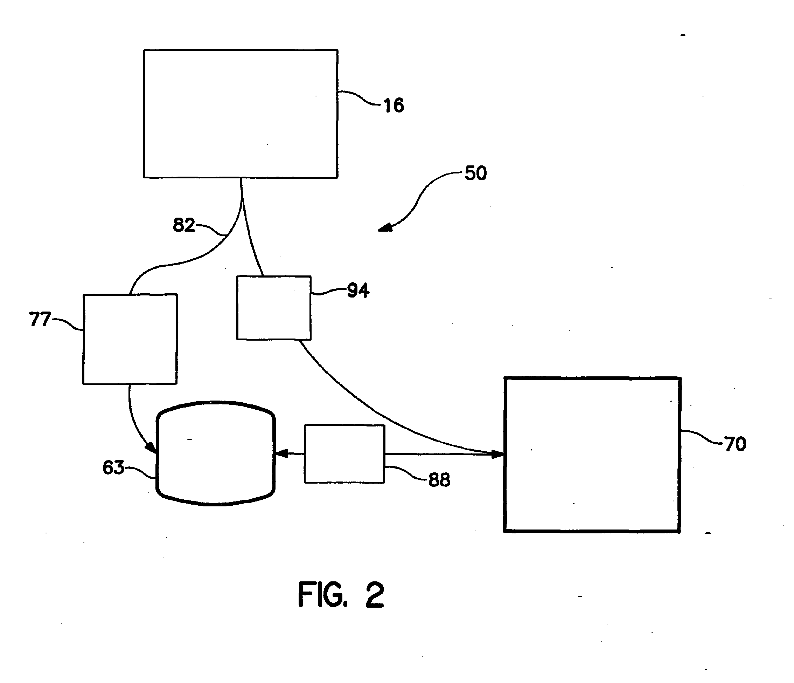

Figure 2 is a schematic representation of a portion of system 50 designed for interacting and managing care for a patient 16 having at least one interactive medical device, such as for example an implanted cardiac, neurologic, infusion or other device. It is recognized that partially implanted or external devices may also be incorporated into the full scope of this disclosure, provided such devices or components are capable of operating in the robust data environment of this invention. Figure 2 illustrates the relation between patient 16, an electronically accessible patient management database 63, and a web-based site 70. Patient management database 63 is configured for receipt, storage, processing, and other transmission and handling of information related to the healthcare status of patients administered by system 50, including patient 16. Physiologic data 77 is uploaded via wireless 82 or other transmission means from devices in or in communication with devices in patient 16, and is of a content designed to provide essential high relevance information regarding all manner of monitored disease etiology. This information is automatically uploaded via path 82 to the database 63, possibly in response to interrogation routines, and subject to power management, disease stage, and other considerations. Data 77 is then registered or otherwise processed as appropriate and transmitted in various forms to at least one web-based site 70, configured to allow access by the patient and others, as will be further discussed below, and as depicted in path 94 with one or more secure sign-in protocols included. It is recognized that patient medical device data 8 8 may also flow from web-based site 70 back to patient management database 63.

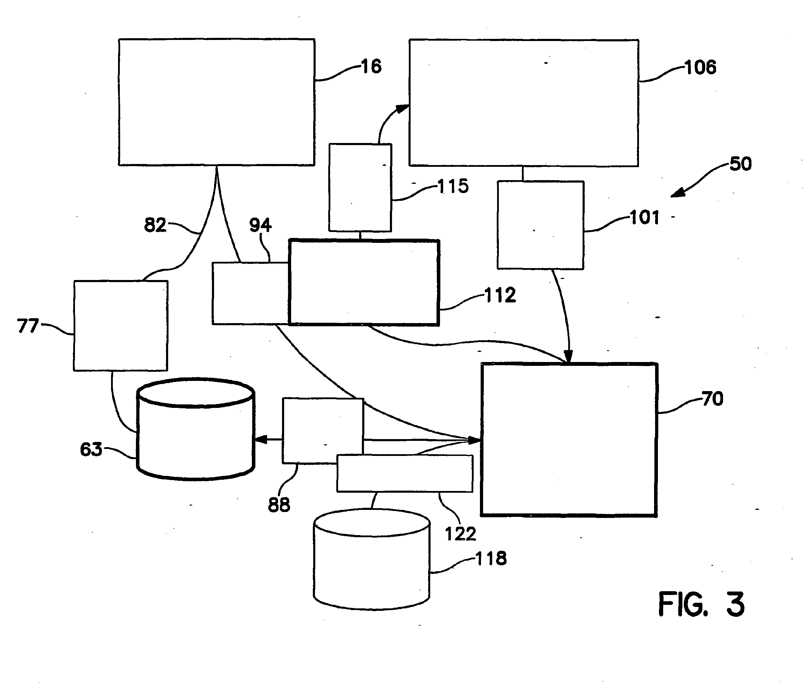

Figure 3 depicts web-based site 70 as a platform which is accessible via secure sign-in 101 by a first medical provider 106 such as a nurse or other type of provider responsible for the care and first stage monitoring of patient 16. A variety of interactions are enabled by this relationship, including use of web-based site 70 as a destination for secure access to provide and receive information pertinent to the care of patient 16, including unique and high relevance physiologic data 77 detected from within one or more patients and transmitted via patient management database 63. In the event of physiologic data 77 containing information meeting certain criteria, such as activating triggers or set points based upon certain analytics, differentials or algorithmic metrics, then the patient management database 63 and the web-based site 70 may be configured to provide an automatic event notification service signal 112 to a display or other data receiving device of one or more medical providers, such as first medical provider 106. In one example, automatic event notification service signal 112 may be pushed via an SMTP.net message, shown for example as signal format 115, to medical provider 106 computer or other display. These displays, and others contemplated herein, may also include an automatic pushed signal via electronic mail, pager, cellular phone, WAP cellular phone, telephone call, facsimile, mobile wireless device, stylus tablet, or others. This automatic event notification service signal 112 may be used to alert provider 106 of a health related signal anomaly or other indication which may require prompt action to assist the patient, and is likely not necessarily a secure message but rather a rapid message is preferable. Accordingly, in one embodiment, the rapid automatic event notification service signal 112 includes a link accessible by the medical provider to rapidly access the data of interest from the web-site 70, and which requires a response by the recipient Such signal 112, and others herein, may also activate an automatic time notated archival feature for later reference for various purposes. As in much of this invention, this path may require a security validation or authentication, depending on system, patient service, and legal requirements. Various biometric or other cipher-like technologies may be utilized, although a smart card or other intelligent and convenient tool may be preferred. For example, one embodiment of a smart card may be able to record medical updates of the patient's health record whenever it is used for access authentication, and thus provide an element of redundancy to the medical record.

First medical provider 106, or others described herein, may be granted secure single sign in rights to facilitate rapid access for the designated individual to the various elements of this system and architecture. This feature may also be transportable as that professional traverses among electronic links in order to ensure connectivity during the response to a detected event, or the access may be a graded access according to an event severity algorithm or other grading technique.

A further feature may include link or other access to a database 118 or other medium having an electronic medical health record 122 of the patient who is the source of the automatic event notification service signal 112. In this manner the patient's pertinent medical history may be presented along with the new data which caused the medical alert, thereby allowing the medical provider with proper perspective and accuracy as to the specific patient. In one embodiment, electronic medical health record 122 is a data rich format such as that known as Extended Markup Language (XML), although various data formats may be utilized to provide the advantages and data contemplated. Moreover, in one embodiment, a single page format is preferred but not required, in order to allow the efficiencies of a template that may be efficiently structured for a single screen display of the most critical information to a final decision maker. This may include a summary, a waveform, a differential diagnosis analysis, coding options, trending graphs, overlays of patient history versus current data, or other high relevance formatted data appropriate to that patient or patient class. Rapid augmenting of that formatted information is also possible, when desired, due to the configuration of this system and the rich data mining capabilities it enables.

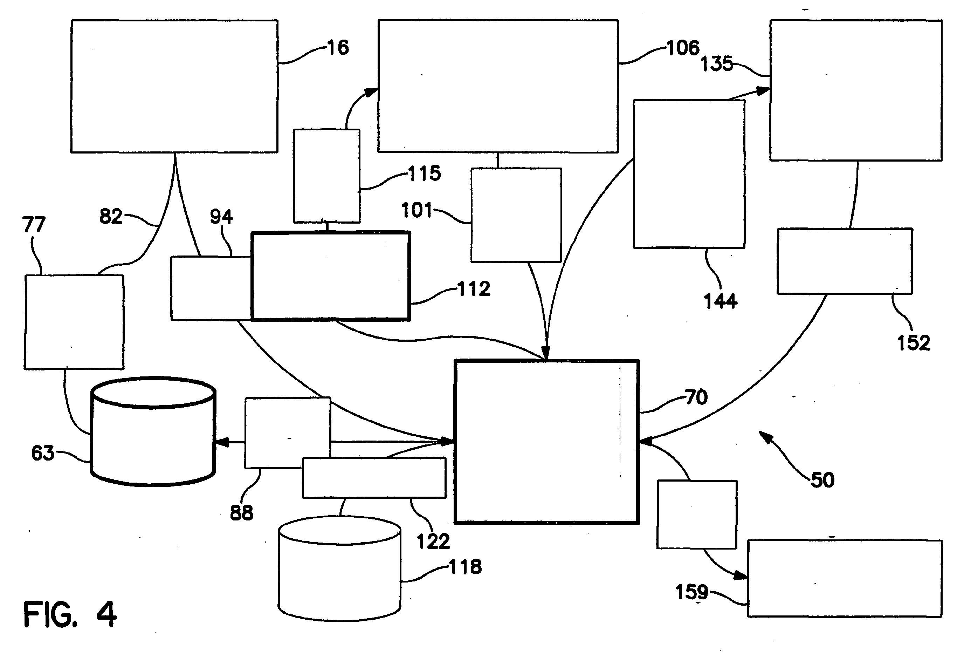

Figure 4 illustrates a further aspect of the invention in which a second medical provider 135 receives either the automatic event notification service signal 112 or, preferably, another form of notification signal 144 which has been generated by another medical provider, such as first medical provider 106. Notification signal 144 is a post-triage type of notification, i.e. after the initial analysis of the high relevance physiologic data has occurred by a first medical provider. The second medical provider may be a more specialized or more highly qualified provider, such as a cardiologist, surgeon or other type of physician. In one embodiment, notification signal may include recommendations or commentary from the first medical provider, and may be in the form of a secure asynchronous XML message, although various formats may be acceptable or preferred, including for example that necessary to enable the above described single page formatted communication. The notification signal 144 may also be formatted for display on a mobile or stationary device of medical provider 135, such as a handheld or other form of personal digital assist device. In this manner, even if the second medical provider is in the process of another activity, it may be possible to have the information observed or otherwise understood from the display and for the second medical provider 135 to direct appropriate medical care in the form of instructions to the patient and other care givers. In one embodiment it is contemplated for the second medical provider 135 to observe the emergent conditions of patient 16 and then dictate a secure voice message 152 (for example, a WAV file attachment or the like) for transmission back to a web-enabled site, such as web-site 70, for merger with the original data and automatic event notification service signal 112 for subsequent transmission to the patient 16. Of course, this information in the form of a combined signal of both the patient's physiologic data and the medical provider's interpretation and care recommendation may also be transmitted to a proxy or other patient advocate 159, as well as to automatic clinic appointment scheduler prompting mechanisms or the like.

Notification of patient 16 that a health-related message is waiting for review may also be sent to the patient advocate 159, such as a close relative of the patient, to ensure that the patient reviews, understands, and complies with the advice of the medical provider(s). This may be a sequence such as receipt of an electronic mail message urging activation of a link to the web-site 70 with automatic routing to message 152. In addition to the various communication options noted herein above, it is possible to have a dedicated channel, optional pop-up alert channel, webTV-like device, or dedicated internet service or portal available to the patient or patient advocate. This may be a service provided by existing or new communication service providers as a fee subscription or other revenue generating mechanism which is able to emerge and possibly block other communications until cleared by the recipient.

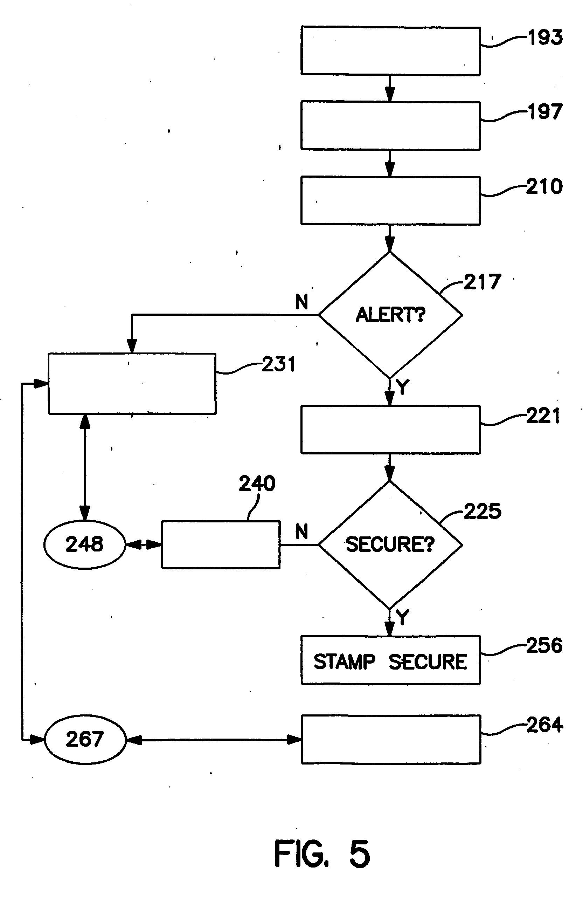

Figure 5 is a high level logic flow chart representative of one embodiment of the computer implemented steps of the invention and the flow of information. It is recognized that alternate flow paths may be implemented within the scope of this invention while still achieving the novelty, inventiveness, and technical contribution that the inventors are merited. Step 193 represents the detect and store features of the medical device that is monitoring the patient or user of the device. Step 197 represents the interrogate function to query and receive physiologic data from the medical device which is likely implanted within a patient. It is recognized that this function step may be obviated for a device which is external in view of different power management options. Step 203 represents the transmission of data to a remotely located patient management database 210 for storage and processing of the received physiologic data. Step 217 represents the analysis and set point computation steps necessary to determine whether the data received comprises particularly high relevance data that necessitates an alert. If an alert is not warranted then at step 219 the data is routed to a web site for instantaneous presentation upon authorized access by either the patient, a proxy or a healthcare professional. If an alert is warranted, then at step 221 a formatting process is implemented, and at step 225 a signal is sent to web-site 231 notification routine causing a non-secure alert signal 240 to be transmitted in a push fashion according to pre-set protocol to healthcare providers. These providers in turn query the web site by rapid return link activation 248 in either a secure or non-secure manner which permits transfer of content rich rendered and formatted signal 256 to the healthcare providers via the web site, and with the signal comprising the high relevance physiologic data necessary for analysis and comment/action by the healthcare providers, leading to transfer to the patient and proxy 264 again via the web site at step 267 with such comment/action recommendations riding thereon.

This invention contemplates various systems and various types of methods to enable substantial economic and medical improvements to the business, art and science of healthcare. It is believed that these innovations and technical contributions provide a powerful combination of features and service advantages in view of the previously stated challenges to real-time effective management of certain classes of patient, such as those with chronic cardiac disease, constant infusion of medicines, neurologic stimulator requirements, or others, and that this has not been accomplished or realistically contemplated before by others.

## Claims

1. An internet-based method for a service to connect a remote patient having an implanted medical device to a database network for medical device data exchange and review comprising:
providing a web-site having a user interface wherein the user interface includes a secure sign-in input to access the database network site;
receiving at the web-site inputs associated with a specific medical device and patient;
confirming the identity of the medical device and the patient;
reviewing data indicative of an event from said implanted medical device;
alerting a medical provider to said event; and
enabling said medical provider to access the database in a single sign-on action to use the service.

2. The method of claim 1 wherein the medical device data exchange and review further comprises at least one of the processes of:
uploading the medical device data into the database; and
accessing the medical device data in the database via a secure web-site.

3. The method of claim 1 wherein said website includes a proxy right access scheme to provide privileged access to a patient's data by friends and family.

## Patentansprüche

1. Internet-basiertes Verfahren für einen Dienst zum Anschließen eines entfernten Patienten mit einem implantierten Medizingerät an ein Datenbanknetz zum Austausch und zur Überprüfung der Medizingerätedaten, umfassend:
Bereitstellen einer Website mit einer Benutzerschnittstelle, wobei die Benutzerschnittstelle eine sichere Anmeldungseingabe für den Internetzugang zu dem Datenbanknetz einschließt;
Empfangen auf dieser Website von Eingaben, die zu einem bestimmten Medizingerät und einem bestimmten Patienten gehören;
Bestätigen der Identität des Medizingerätes und des Patienten;
Überprüfen von Daten von diesem implantierten Medizingerät, die ein Ereignis anzeigen;
Alarmieren eines medizinischen Dienstanbieters über dieses Ereignis; und
Befähigen dieses medizinischen Dienstanbieters, auf die Datenbank durch einen einzigen Anmeldungsakt zuzugreifen, um den Dienst in Anspruch zu nehmen.

2. Verfahren nach Anspruch 1, wobei der Austausch und die Überprüfung von Medizingerätedaten weiterhin zumindest einen der folgenden Vorgänge umfassen:
Hochladen der Medizingerätedaten in die Datenbank; und
Zugriff auf die Medizingerätedaten in der Datenbank über eine sichere Website.

3. Verfahren nach Anspruch 1, wobei die Website ein Vertretungsrecht-Zugangsschema einschließt, um für Freunde und Familienangehörige einen bevorrechtigten Zugang zu den Daten eines Patienten bereitzustellen.

## Revendications

1. Procédé utilisant l'Internet pour un service pour relier un patient à distance ayant un dispositif médical implanté à un réseau de bases de données pour un échange et un examen de données de dispositif médical, comprenant les opérations consistant à :
- proposer un site internet ayant une interface utilisateur, l'interface utilisateur comprenant une entrée d'ouverture de session sécurisée pour accéder au site de réseau de bases de données ;
- recevoir au site internet des entrées associées à un dispositif médical et un patient spécifiques ;
- confirmer l'identité du dispositif médical et du patient ;
- examiner des données indicatives d'un événement à partir dudit dispositif médical implanté ;
- alerter le fournisseur de service médical dudit événement ; et
- permettre audit fournisseur de service médical d'accéder à la base de données en une seule action d'ouverture de session pour utiliser le service.

2. Procédé selon la revendication 1, dans lequel l'échange et l'examen des données de dispositif médical comprend en outre au moins l'un des procédés consistant à :
- télécharger vers l'amont les données de dispositif médical dans la base de données ; et
- accéder aux données de dispositif médical dans la base de données par l'intermédiaire d'un site internet sécurisé.

3. Procédé selon la revendication 1, dans lequel ledit site internet comprend un schéma de droits d'accès à serveur mandataire pour fournir un accès privilégié aux données de patient par les amis et la famille.
